# EUROPEAN PATENT SPECIFICATION

(11) **EP 1 955 684 B1**
(45) Date of publication and mention of the grant of the patent: **01.07.2009**
(21) Application number: 08100875.7
(22) Date of filing: 24.01.2008
(51) Int. Cl.: A61F 9/007

(54) **Surgical probe**
Chirurgische Sonde
Sonde chirurgicale

(30) Priority: 12.02.2007 US 673895
(43) Date of publication of application: 13.08.2008
(73) Proprietor: Alcon, Inc., 6331 Hünenberg (CH)
(72) Inventor: Attinger, Jürg, 8260, Stein am Rhein (CH)
(74) Representative: Hanna, Peter William Derek

(56) References cited:
- EP-A- 0 769 279
- EP-A- 1 632 205
- EP-A- 1 782 781
- US-A- 4 869 715

## Description

### Background of the Invention

This invention relates generally to the field of microsurgery and, more particularly, to ophthalmic microsurgery.

Current vitreoretinal techniques in which surgical instruments are inserted into the eye require the dissection of the conjunctiva and the creation of pars plana scleral incisions through the sclera. The dissection of the conjunctiva typically involves pulling back the conjunctiva about the eye so as to expose large areas of the sclera and the clipping or securing of the conjunctiva in that pulled back state. Following the creation of the incisions, surgical instruments are passed through these incisions and the inserted instruments are observed through the pupil using a microscope and corrective optics. These instruments are used to manipulate and/or dissect retinal tissues within the eye as well as to implement the specific retinal treatment technique (e.g., photocoagulation). Prior art scleral incisions created for vitreoretinal surgery are made large enough to accommodate the required instruments, the inserted portions being typically 19 or 20 gauge (approximately 1 mm) in diameter. After completing the specific treatment procedure, the inserted instruments are removed from the incisions in the sclera. Because the incisions through the sclera are large enough to pass 19 or 20 gauge instruments, the incisions are typically too large to self-seal. Thus, the incisions must be sutured shut. Following the suturing of the scleral incisions, the surgical personnel reposition the conjunctiva in its normal position and reattach the free end(s) of the conjunctiva to the eye using sutures. While such methods and techniques have proven to be effective in the treatment of vitreoretinal disease, there is a strong motivation to move away from procedures requiring sutures and instead look to greatly simplified sutureless procedures. Therefore, recently surgical instruments have been miniaturized so that the cannulae or shafts of the instruments are on the order of 23 or 25 gauge. Such thin shafts are bent easily, particularly as they are manipulated within very tight wounds.

Therefore, a need continues to exist for a thin gauge probe that more easily resists bending during use.

### Brief Summary of the Invention

The present invention improves upon the prior art by providing a thin gauge surgical probe having a retractable reinforcing sleeve, in accordance with claims which follow. When the probe is inserted into the eye, through the trocar cannula, the trocar cannula becomes removably attached to the distal end of the sleeve is a trocar cannula, and acts as a stiffening device.

Accordingly, one objective of the present invention is to provide a thin gauge surgical probe.

Another objective of the present invention is to provide a thin gauge surgical probe that resists bending.

Yet another objective of the present invention is to provide a thin gauge surgical probe having a retractable reinforcing sleeve with a removably attached trocar cannula.

Another objective of the present invention is to provide a thin gauge surgical probe having a retractable reinforcing sleeve.

These and other advantages and objectives of the present invention will become apparent from the detailed description and claims that follow.

### Brief Description of the Drawings

FIG. 1 is a perspective view of the probe of the present invention.
FIG. 2 is an enlarged cross-sectional view of the probe of the present invention.
FIG. 3 is an enlarged cross-sectional view of the trocar cannula that may be used with the present invention taken at circle 3 in FIG. 2.

### Detailed Description of the Preferred Embodiments

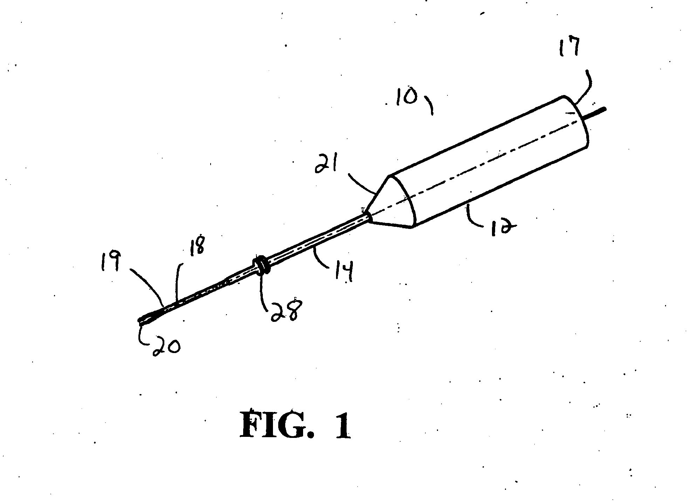

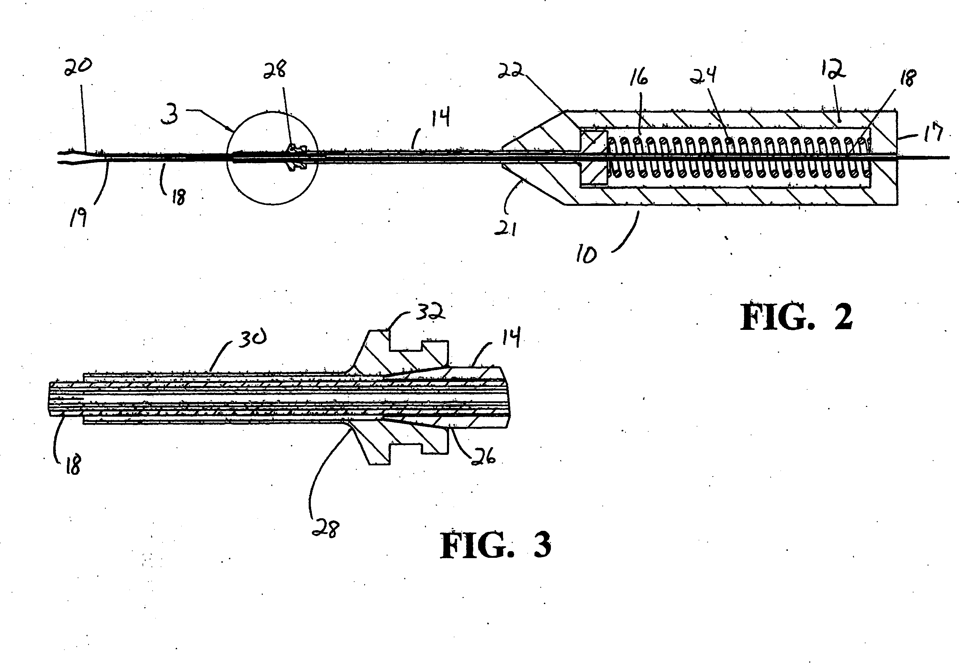

As best seen in FIGS. 1 and 2, probe 10 consists of probe handle or body 12 and cannula 14. Body 12 may be made of any suitable material, such as stainless steel, titanium or plastic. Body is generally hollow, having interior cavity 16. Extending through body 12 and cannula 14 and fixed to proximal end 17 of body 12 is tool shaft 18. Distal end 19 of shaft 18 opposite proximal end 17 of body 12 contains tool 20. Tool 20 may be any suitable tool such as a forceps or scissors.

Cannula 14 generally is made of thin walled stainless steel or titanium tubing with an outside diameter of 20, 23 or 25 gauge or smaller. Cannula 14 movably extends through distal end 21 of body 12 and retained within body 12 by sleeve 22. Sleeve 22 reciprocates within interior cavity 16 and is urged toward distal end 21 of body 12 by spring 24.

Attached to distal end 26 of cannula 14 opposite spring 24 is trocar cannula 28. Trocar cannula 28 generally consists of cannula 30 that is attached to or integrally formed with hub 32. Trocar cannula 28 may be made from any suitable thermoplastic or metal, such as titanium or stainless steel. Hub 32 is sized to engage, frictionally and removably, or form the closure for and be removable from, distal end 26 of cannula 14.

As seen in FIG. 2, in use, tool 20, shaft 18 and cannula 30 are inserted into a surgical incision until hub 32 contacted the region around the wound. Tool 20 may be pushed deeper within the wound by further pushing of body 12. As cannula 14 slides about shaft 18, pushing on body 12 causes trocar cannula 28 to push cannula 14 against sleeve 22, thereby exposing more of shaft 18 within the wound. As probe 10 is withdrawn from the wound, spring 24 pushes on sleeve 22, thereby causing cannula 14 to slide distally over shaft 18.

In addition, as trocar cannula is releasable from cannula 14, probe 10 may be disengaged from trocar cannula 28 and tool 20 removed from the surgical site so that another tool (not shown) may be inserted into the incision through trocar cannula 28.

This description is given for purposes of illustration and explanation. It will be apparent to those skilled in the relevant art that changes and modifications may be made 25 to the invention described above without departing from its scope.

## Claims

1. A surgical probe (10), comprising:
a) a body (12) having a hollow interior cavity (16);
b) a shaft (18), the shaft extending through the body and fixed to a proximal end (17) of the body;
c) a tool (20) fixed to the shaft opposite the proximal end of the body;
d) a cannula (14) coaxially mounted over the shaft, a proximal end of the cannula penetrating into the hollow interior cavity of the body;
e) a sleeve (22) slidably received over the shaft and contained within the hollow interior of the body, the sleeve attached to the proximal end of the cannula;
f) a spring (24) biasing the sleeve distally; and
g) a trocar cannula (28) slidably received on the shaft and removably attached to a distal end (26) of the cannula.

2. The probe of claim 1 wherein the spring (24) is mounted within the hollow interior cavity (16) of the body (12).

## Patentansprüche

1. Chirurgische Sonde (10), mit:
a) einem Körper (12), der im Innern einen hohlen Hohlraum (16) hat;
b) einem Schaft (18), wobei sich der Schaft durch den Körper erstreckt und am proximalen Ende (17) des Körpers befestigt ist;
c) einem Werkzeug (20), das am Schaft gegenüber dem proximalen Ende des Körpers befestigt ist;
d) einer Kanüle (14), die koaxial über dem Schaft angebracht ist, wobei das proximale Ende der Kanüle in den hohlen Hohlraum im Innern des Körpers eindringt;
e) einer Hülse (22), die gleitbar über dem Schaft aufgenommen und innerhalb des hohlen Hohlraums des Körpers enthalten ist, wobei die Hülse am proximalen Ende der Kanüle angebracht ist;
f) einer Feder (24), die die Hülse distal vorspannt; und
g) einer Trokar-Kanüle (28), die gleitbar auf dem Schaft aufgenommen und abnehmbar am distalen Ende der Kanüle (26) der Kanüle angebracht ist.

2. Sonde nach Anspruch 1, bei der die Feder (24) innerhalb des hohlen Hohlraums (16) des Körpers (12) eingebaut ist.

## Revendications

1. Sonde chirurgicale (10), comprenant :
a) un corps (12) ayant une cavité intérieure creuse (16) ;
b) un arbre (18), l'arbre s'étendant à travers le corps et étant fixé sur une extrémité proximale (17) du corps ;
c) un outil (5) fixé sur l'arbre opposé à l'extrémité proximale du corps ;
d) une canule (14) montée coaxialement sur l'arbre, une extrémité proximale de la canule pénétrant dans la cavité intérieure creuse du corps ;
e) un manchon (22) reçu de manière coulissante sur l'arbre, et contenu dans l'intérieur creux du corps, le manchon étant fixé sur l'extrémité proximale de la canule ;
f) un ressort (24) rappelant le manchon distalement ; et
g) une canule de trocart (28) reçue de manière coulissante sur l'arbre, et fixée de manière amovible sur une extrémité distale (26) de la canule.

2. Sonde selon la revendication 1, dans laquelle le ressort (24) est monté dans la cavité intérieure creuse (16) du corps (12).
